# EUROPEAN PATENT SPECIFICATION

(11) **EP 4 622 698 B1**
(45) Date of publication and mention of the grant of the patent: **16.09.2026**
(21) Application number: 23810365.9
(22) Date of filing: 23.11.2023
(51) Int. Cl.: A61M 16/14

(54) **ADMINISTERING AN AEROSOL TO A PATIENT**
VERABREICHUNG EINES AEROSOLS AN EINEN PATIENTEN
ADMINISTRATION D'UN AÉROSOL À UN PATIENT

(30) Priority: 24.11.2022 EP 22209377
(43) Date of publication of application: 01.10.2025
(73) Proprietor: Fraunhofer-Gesellschaft zur Förderung der angewandten Forschung e.V., 80686 München (DE)
(72) Inventor: WIEGANDT, Felix, 80686 München (DE); POHLMANN, Gerhard, 80686 München (DE)
(74) Representative: Altmann Stößel Dick Patentanwälte PartG mbB
(86) International application number: PCT/EP2023/082865
(87) International publication number: WO 2024/110588

(56) References cited:
- WO-A1-2020/088984
- DE-A1- 102008 050 218
- DE-A1- 102020 120 036
- US-B2- 10 987 474
- BENNY FENG ET AL: "A Novel In-Line Delivery System to Administer Dry Powder Mannitol to Mechanically Ventilated Patients", JOURNAL OF AEROSOL MEDICINE AND PULMONARY DRUG DELIVERY, vol. 30, no. 2, 1 April 2017 (2017-04-01), US, pages 100 - 107, XP055577613, ISSN: 1941-2711, DOI: 10.1089/jamp.2016.1320

## Description

### Field of the invention

The present invention relates to an apparatus for administering an aerosol to a patient and to an aerosol generating device configured for generating an aerosol stream. In particular, the apparatus may be designed for providing a defined aerosol concentration preferably a high aerosol concentration, to the patient, especially but not limited to a preterm infant, a newborn, a child or an adult.

### Related art

Pulmonary diseases, such as asthma or chronic obstructive pulmonary disease (COPD), are, usually, treated by inhalation of corresponding aerosols. Further, efforts exist to deliver a surfactant in form of an aerosol during ventilation to a preterm infant having a surfactant deficiency, especially since this form of therapy is non-invasive and, thus, less detrimental for the preterm infant in contrast to the standard therapy using invasive instillation of a surfactant suspension.

Aerosols may be continuously generated and administered to a patient without breath synchronized aerosol release. However, a share of a continuously delivered aerosolized drug may be lost and may, thus, show no medical benefit. As further demonstrated by Longest, P.W.; Azimi, M.; Hindle, M., Optimal Delivery of Aerosols to Infants During Mechanical Ventilation, Journal of Aerosol Medicine and Pulmonary Drug Delivery 2014, 27, 371-385, doi:10.1089/jamp.2013.1077, only aerosol delivered during the first half of an inhalation phase reaches the periphery of the alveoli. Further, a local pulmonary drug requirement for an inhaled application of a surfactant considerably may exceed that of other inhaled application of aerosols for pulmonary diseases.

Consequently, a breath-triggered drug release is of high interest. When breath is detected, the aerosol must be released accordingly, i.e. over a defined period of time, such as a short or long aerosol boli. However, known approaches for breathing detecting during respiratory support or independent breathing and for aerosol release offer various problems and shortcomings.

Known respiration monitoring devices work via volume stream or pressure sensors, which are configured to determine an inhalation flow at the patient interface. As a result, a drug release can only occur after a delay, i.e. not directly at the start of inhalation. Accordingly, a pressurized metered-dose inhaler can be integrated in combination with a spacer into a ventilation circuit. However, a pharmaceutical preparation is only released via active actuation of the inhaler by the patient or a further person. As an alternative, an aerosol generating device can be coupled into the ventilation circuit by using an adapter.

Triggering of the aerosol release can be based on a detection of a pressure change in the ventilation tube caused by breathing, whereupon the aerosol generation is activated or deactivated. However, the drug is released only after a corresponding pressure change has been registered which occurs with a delay. As an alternative, the drug release can be controlled by using a pressure measurement at a mouthpiece, wherein an evaluation algorithm may consider an averaging over consecutive breaths.

US 10 987 474 B2 discloses an aerosol delivery system that includes an aerosol generator that aerosolizes a fluid for delivery to a patient as a patient inhales. The aerosol delivery system includes a pump coupled to the aerosol generator that pumps the fluid to the aerosol generator, and a breath sensor that emits a signal as the patient breathes. A controller couples to the aerosol generator, the pump, and the breath sensor. **In** operation, the controller receives the signal from the breath sensor, controls a flow of fluid to the aerosol generator in response to the signal, and controls the aerosol generator to start aerosolizing the fluid before the patient inhales.

US 2021/0283345 A1 discloses an aerosol delivery system having an aerosol generator that aerosolizes a fluid for delivery to a patient as a patient inhales. The aerosol delivery system includes a pump coupled to the aerosol generator that pumps the fluid to the aerosol generator, and a breath sensor that emits a signal as the patient breathes. A controller couples to the aerosol generator, the pump, and the breath sensor. **In** operation, the controller receives the signal from the breath sensor, controls a flow of fluid to the aerosol generator in response to the signal, and controls the aerosol generator to start aerosolizing the fluid before the patient inhales.

WO 2020/243107 A1 and US 2020/368457 A1 disclose a method of delivering aerosolized surfactant to an infant that includes interfacing an aerosolization device with an airway of an infant and aerosolizing, using the aerosolization device, a volume of surfactant into particles having a mass mean aerodynamic diameter of less than about 3 µm at a rate of at least 0.1 *mL*/*min.* The surfactant is aerosolized within about 1 to 8 cm from a patient interface. Aerosol is generated for up to approximately 80% or each inspiration. The method also includes delivering the aerosolized surfactant to the infant's airway.

WO 2019/115771 A1 discloses a nebulizer system comprising a nebulizer for assisted breathing devices. The nebulizer comprises a body having a first connection for connecting the nebulizer to the assisted breathing device and a second connection for connecting the nebulizer to a patient, particularly a neonate, wherein the body forms a flow channel from the first connection to the second connection, and a nebulizing device for nebulizing a liquid. The nebulizing device is arranged in the flow channel between the first connection and the second connection. The nebulizer is configured for being adapted to an oral and/or nasal communication element, such as nasal prongs, a nose mask, a face mask or a mouthpiece. Further, the the disclosure imentien- relates to a holding system for holding a nebulizer or a nebulizer system.

WO 2020/079055 A1 discloses methods, devices and compositions for use in inhalation therapy of neonates, infants or children, suffering from a disease, optionally a pulmonary disease, such as asthma, by which high amounts of inhaled drugs are directed to the small airways of the peripheral lungs of neonates, infants and children using slow, controlled flow rates and pre-set inhalation volumes. A novel inhalation device tailored for use in neonates, infants and children and adapted to provide said slow, controlled flow rates with a simplified jet nebulizer set-up is disclosed, together with kits comprising the device.

However, most known approaches are based on predictions that are usually inappropriate since a preterm infant shows an irregular respiration, such that the aerosol cannot be generated and released in an optimal fashion. Owing to an inherent dead space volume, such devices are often not suitable for being used with a preterm infant.

US 2019/0247595 A1 discloses a system for respiration-controlled application of aerosol in powder form during artificial respiration or assisted respiration of a patient including an interface contacting the patient's respiratory tract, a unit for generating a respiratory gas flow, at least one inspiration line through gas flow is conducted to the interface, an aerosol generator, at least one aerosol line through which the generated aerosol is conducted from the aerosol generator to the interface, and a respiration sensor that detects the patient's respiration signal. A valve in the at least one aerosol line is controlled based on the detected respiratory signal. An intermediate store for generated aerosol in powder form is arranged between the valve and the aerosol generator. The gas flow has a first pressure that is higher than or equal to ambient pressure and the aerosol has a second pressure that is higher than or equal to the first pressure.

WO 2012/020004 A1 discloses a device for connection of the airways of a patient on ventilatory support with a source of a breathing gas and a source of an aerosol. Said device comprises a contact component which is adapted to be positioned in contact with the airways of the patient and which comprises: one or two tubes each comprising a lumen through which breathing gas and aerosol can be delivered to the airways of the patient, a mixing lumen which is in fluid connection with the lumen or lumens of the tube or the two tubes and having a longitudinal axis extending substantially perpendicularly to longitudinal axis or axes of the lumen or lumens and a port through which aerosol can be introduced into said mixing lumen and arranged such that the aerosol and a breathing gas can be mixed in the mixing lumen.

Wiegandt, F.C.; Biegger, D.; Fast, J.F.; Matusiak, G.; Mazela, J.; Ortmaier, T.; Doll, T.; Dietzel, A.; Bohnhorst, B.; Pohlmann, G., Detection of Breathing Movements of Preterm Neonates by Recording Their Abdominal Movements with a Time-of-Flight Camera, Pharmaceutics 2021, 13, doi:10.3390/pharmaceutics 13050721, describe that the initiation of the patient's inspiration needs to be detected in order to deliver an aerosolized drug in a breath-triggered manner. Respiratory sensors, especially when invasive, can be tested on preterm neonates only with great effort due to clinical and ethical hurdles. Therefore, a physiological model is highly desirable to validate these sensors. For developing such a system, abdominal movement data of preterm neonates are required. The authors recorded time sequences of five preterm neonates' abdominal movements with a time-of-flight camera and successfully extracted various respirations patterns and respiratory parameters. Several characteristic respiration patterns, such as forced breathing, sighing, apnea and crying, were identified from the movement data. Respiratory parameters, such as duration of inspiration and expiration, as well as respiratory rate and breathing movement over time, were also extracted. They demonstrated that respiratory parameters of preterm neonates can be determined without contact. Therefore, such a system can be used for breathing detection to provide a trigger signal for breath-triggered drug release systems.

Koch, E.; Dietzel, A, Skin attachable flexible sensor array for respiratory monitoring, Sensors and Actuators A: Physical 2016, 250, 138-144, doi:10.1016/j.sna.2016.09.020, describe a 6 × 6 sensor array for curvature sensing in a format of a thin flexible polyimide foil. The sensor foil is to be used for respiratory monitoring of premature infants by directly attaching it to the skin for measuring the body deformations caused by breathing. Sensor signals shall in future be used not only to trigger the respiration device but also to provide time dependent body surface reconstruction as a diagnostic tool. One single sensor element consists of four gold strain gauges in a Wheatstone bridge configuration. For suppressing sensor response to foil stretching and for increasing bending sensitivity, they introduced a double-sided sensor design with strain gauges on both surfaces of the thin foil leading to a 170% higher sensitivity than a one-sided sensor design. Double sided sensor elements with different orientations are arranged in an alternating pattern across the array allowing to fully and unambiguously determine the bending vector utilizing plausibility considerations on basis of signals from neighboring elements.

DE 10 2020 120 036 A1 discloses a method for operating a heating device of a system for controlled respiratory hyperthermia treatment on the human or animal organism, wherein ambient air is drawn into the heating device as inspiratory air via a first air inlet and a first valve, in particular a controllable valve or a check valve, into the heating device, and is heated by a heating unit and a heat exchanger thermally connected to the heating unit, and is fed via an inspiratory air breathing tube, which can be heated, in particular for further heating, and controlled by a temperature sensor, to a breathing mask. The inspiratory air is temporarily stored in the heating device in a reservoir downstream of the heat exchanger and fed into the inspiratory air breathing tube by opening a controllable second valve downstream of the reservoir, when a pressure sensor on the breathing mask measures a negative pressure.

DE 10 2008 050 218 A1 discloses a system having a respirator connected with an ex-vivo rabbit lung over an inspiration canal and an expiration canal. A nebulizer is provided in the inspiration canal for nebulizing an inhalable material. A controller attached with the nebulizer operates the nebulizer only at beginning or during an inspiration phase and stops the nebulizer during termination of the inspiration phase. A pressure sensor is arranged in the inspiration canal between the respirator and the nebulizer, and connected with the controller.

WO 2020/088984 A1 discloses an apparatus to administer drugs to mechanically ventilated patients, comprising: a mechanical ventilator, an artificial airway to be associated to a patient and a ventilation circuit connecting the mechanical ventilator to the artificial airway. The ventilation circuit comprises: an inspiratory line, a dry powder inhaler disposed in line on the inspiratory line and a connector operatively connected to the dry powder inhaler and to the inspiratory line. The connector comprises: a first duct facing an outlet port of the dry powder inhaler and connected or configured to be connected to a tube section of the inspiratory line placed downstream the dry powder inhaler; a second duct facing an air inlet port of the dry powder inhaler and connected or configured to be connected to a tube section of the inspiratory line placed upstream the dry powder inhaler.

US 10 987 474 B2 discloses an aerosol delivery system that includes an aerosol generator that aerosolizes a fluid for delivery to a patient as a patient inhales. The aerosol delivery system includes a pump coupled to the aerosol generator that pumps the fluid to the aerosol generator, and a breath sensor that emits a signal as the patient breathes. A controller couples to the aerosol generator, the pump, and the breath sensor. In operation, the controller receives the signal from the breath sensor, controls a flow of fluid to the aerosol generator in response to the signal, and controls the aerosol generator to start aerosolizing the fluid before the patient inhales.

Benny Feng et al: "A Novel In-Line Delivery System to Administer Dry Powder Mannitol to Mechanically Ventilated Patients", Journal of Aerosol Medicine and Pulmonary Drug Delivery 30(2), 2017, pages 100-107 describes an inspiratory line from a ventilator, which was split by using a three-way valve into two parallel lines where one contains a humidifier for normal breathing cycle and the other line contains a dry powder inhaler. The inspiratory air went through the dry powder line and aerosolized the mannitol powder only when its administration to a patient is required.

### Problem to be solved

It is therefore an objective of the present invention to provide an apparatus for administering an aerosol to a patient and an aerosol generating device configured for generating an aerosol stream, which at least partially avoid the problems and shortcomings of the known approaches.

It is particularly desirable that the apparatus and the methods would be able to administer a large portion of a pharmaceutical preparation comprised by an aerosol to a patient, especially but not limited to a preterm infant, a newborn, a child or an adult, specifically in order to ensure that an envisaged amount of the pharmaceutical preparation may actually be provided to the patient in a desired concentration and may not be diluted and/or distributed elsewhere. It is further desirable to reduce or, preferably, to avoid a delay in administering the pharmaceutical preparation to the patient. It is still further desirable to be able to administer a pharmaceutical preparation to one or more predefined targets in central or peripheral areas of the lung.

### Summary of the invention

This problem is solved by an apparatus for administering an aerosol to a patient and an aerosol generating device configured for generating an aerosol stream having the features of the independent claim. Preferred embodiments are disclosed by the dependent claims.

In a first aspect, the present invention refers to an aerosol generating device configured for generating an aerosol stream, according to claim 1.

Herein, the conducting element may, preferably, comprise at least one of a patient interface or an aerosol valve as disclosed below in more detail. However, using a tube may also be feasible. In addition, the aerosol generating device may comprise at least one further element, in particular a breathing filter as disclosed elsewhere herein. However, using at least one different further element may also be conceivable.

As generally used, the term "aerosol" refers to an aerosolizable material that comprises solid or liquid particles of a substance which are suspended in a gas phase, wherein the particles may, in particular, be or comprise particles of a pharmaceutical preparation, such as but not limited to a lung surfactant. For converting the particles into this state, an aerosolizable material, i.e. a powder or a liquid solution, is treated in an aerosol generating element, in particular by using a powder generator or a nebulizer, especially vibrating meshes or ultrasonic waves, in order to entrain the solid or liquid particles into a gas stream of a carrier gas, such as a respiratory gas. In this state, the particles are, preferably, distributed across the entire volume of the carrier gas, in particular, in a uniform and finely dispersed form. As a result, the aerosol is provided as an "aerosol stream" in which the solid or liquid aerosol particles are borne or carried by the carrier gas stream. Further, the term "administering" or any grammatical variation thereof refers to a process of providing a controlled application of the respiratory gases and the aerosol comprised hereby, in particular, by carrying a predefined amount of the pharmaceutical preparation as comprised by the humidified aerosol per period of time to the patient. As used herein, the term "respiratory gases" refers to a gas mixture which comprises a composition being suitable for the ventilation of a patient, in particular, air or oxygen-enriched air.

Further, the term "ventilatory circuit" refers to a device configured for a ventilation of the respiratory gases as provided by a ventilator to the patient and from the patient back to the ventilator, hereby excluding the respiratory tracks of the patient. As further used herein, the term "patient" refers to a human being of any age, in particular, including a preterm infant, a newborn, a child, or an adult. Further, the term "ventilation" relates to a process of accomplishing a movement of the respiratory gases, in particular, via alternating steps of inhalation and exhalation. In contrast to healthy breathing patients who are capable of performing the breathing without any additional aids, patients who are subject to mechanical ventilation or positive pressure ventilation, require the respiratory gases at least partially to be provided from the ventilator via the ventilatory circuit. As used herein, the term "patient interface" refers to a device being configured for providing a connection between the ventilatory circuit and the respiratory track of the patient which is therefore, in general, located adjacent to the patient. For this purpose, the patient interface may be integrated into, or attached to, the ventilatory circuit, wherein the ventilatory circuit may, in general, comprise a ventilator and tubes adapted for guiding gases from the ventilator to a patient interface and back. In particular, a mouthpiece, a breathing mask, a nasal cannula, a nasal prong or a tracheal cannula may be part of the patient interface or attachable thereto. However, further arrangements may also be feasible.

According to the present invention, the aerosol generating device comprises an air inlet that is configured for receiving a portion of breathing air from the respiratory circuit to be provided to an aerosol generating element as further comprised by the aerosol generating device. Herein, the aerosol generating element is configured for generating aerosol particles and introducing them into the carrier gas. As used herein, the term "aerosol generating element" refers to an element which is designated for converting an aerosolizable material, i.e. a powder or a liquid solution, into an aerosol, in particular by using a powder generator or a nebulizer, especially vibrating meshes or ultrasonic waves, in order to entrain the solid or liquid particles into the gas stream of the carrier gas, such as the respiratory gas. For this purpose, the aerosol generating element may, preferably, be selected from at least one of a nebulizer or a powder generator. Further, the aerosol generating device comprises an aerosol tube which is configured for providing the aerosol stream that comprises the aerosol particles through a conducting element, which may, preferably, comprise at least one of a patient interface or an aerosol valve, to the patient.

According to the present invention, the aerosol generating device further comprises a pressure modulation element. As used herein, the term "pressure modulation element" refers to an element which is configured for modulating a pressure, in particular for providing an additional pressure within the aerosol stream. Preferably, the pressure modulation element may be selected from at least one of a ventilator, a fan, a pump, a mechanical element or an electromechanical element configured for this purpose. In particular, the additional pressure may be used for improving a manner of guiding of the aerosol stream to the patient through a conducting element, which may, preferably, comprise at least one of the patient interface or the aerosol valve. According to the invention, a pressure prevailing at the aerosol tube, when the aerosol is administered to the patient, in particular when the aerosol valve is in an open position, exceeds a further pressure prevailing at the air inlet by at least 0.1 mbar, preferably by at least 0.05 mbar, more preferred by at least 0.02 mbar, especially by at least 0.01 mbar.

In an additionally preferred embodiment, the pressure modulation element may, further, be configured for limiting the additional pressure within the aerosol generating device to an additional peak pressure. Herein, the additional peak pressure may assume a value of at maximum 20 mbar or 10 mbar, preferably 5 mbar, more preferred 2 mbar, especially 1 mbar, or below. Limiting the additional peak pressure in the patient interface, when the aerosol is administered to the patient, in particular when the aerosol valve is in an open position, can contribute to avoiding a damage to the patient by a too high pressure. In this additionally preferred embodiment, a volume flow of 0.01 *L*/*min* to 3 *L*/*min,* on average approx. *1 L*/*min,* can, preferably, be generated by the pressure modulation element. Herein the low volume flow of 0.01 *L*/*min* to 3 *L*/*min,* on average approx. *1 L*/*min,* compared to the volume flow in the ventilation circuit, enables a high, virtually undiluted aerosol concentration to be transported directly to the patient.

In a further particular embodiment, the aerosol generating device may, additionally, comprise a breathing filter that may be placed at an upstream location with respect to the aerosol generating element. As generally used, the term "breathing filter" refers to a filtering element which is configured for removing at least one interfering substance from the breathing air to be provided to the aerosol generating element.

The aerosol generating device according to the present invention is used in a bypass arrangement with respect to the respiratory circuit. As generally used, the term "bypass" refers to an arrangement of flows, wherein a flow portion is removed from a main flow and reintroduced into the main flow after a separate treatment. Herein, a portion of the breathing air is removed from the ventilatory circuit, enriched with aerosol, and reintroduced into the ventilatory circuit for administering the aerosol-enriched breathing air to the patient. Accordingly, the aerosol generating device according to the present invention differs from the aerosol generator as disclosed in US 10 987 474 B2, which is silent about the bypass arrangement according to the present invention. The aerosol generating device according to the present invention exhibits the advantage that the bypass arrangement is configured for transporting a high, virtually undiluted aerosol concentration directly into the patient interface and, preferably, via a mouthpiece, a breathing mask, a nasal cannula, a nasal prong or a tracheal cannula to the patient.

As a further advantage, the aerosol generating device according to the present invention is not integrated into the patient interface; rather the aerosol stream is supplied directly to the patient interface, independently of the respiratory gas flow of the ventilatory circuit. This arrangement minimizes an overall weight of the patient interface. Thereby, damages at the noses and/mouths of the preterm infants, newborns, and children can be reduced or avoided. In addition, the direct supply of the aerosol stream to the patient interface does not generate an additional dead space volume, in contrast to a direct aerosolization in the patient interface, such as disclosed in WO 2020/243107 A1, WO 2020/079055 A1, or WO 2019/115771 A1. Rather, the dead space volume according to the present invention only corresponds to a volume of the patient interface and, in particular, of the mouthpiece, the breathing mask, the nasal cannula, the nasal prong or the tracheal cannula.

It is particularly advantageous that a maximum pressure on entering the patient interface can be limited a priori by using the pressure modulation element, thus ruling out patient injury a priori. In addition, the aerosol concentration can be varied by controlling the strength of the bypass arrangement volume flow depending on the pressure modulation element, so that a higher aerosol concentration may be achieved by using a lower volume flow. In addition, no need exists to precisely record and control the pressure and volume flow compared to known devices in which air is fed in from a compressed air network. As a result of these advantages, the aerosol generating device according to the present invention can be used for both liquid and powdered medication to be provided to a patient of any age, including a preterm infant, a newborn, a child or an adult.

In particular, the aerosol generating device according to the present invention may be configured to operate independently of the ventilation system and/or the ventilatory circuit, especially independent from any parameters used for controlling the ventilation system and/or the ventilatory circuit. As a result, the aerosol generating device may be adapted to deliver a controlled aerosol stream to the patient without reliance on, or synchronization with, operational settings or cycles of the ventilation system, thereby ensuring consistent and effective aerosol delivery irrespective of the ventilation system's state or mode of operation. Further, the aerosol generating device may be configured to variably adjust the aerosol concentration in response to changes in volume flow, facilitated by the pressure modulation element, which is configured to modulate the additional pressure within the aerosol stream for delivery to the patient through a conducting element. Further, the aerosol generating device can be engineered to operate without a need for an external pressure sensor or control unit, particularly by utilizing the bypass arrangement to harness the pressure from the ventilatory circuit and the pressure modulation element to minimally increase this pressure, thereby enabling efficient aerosol delivery without the complexity of additional pressure monitoring or controlling components.

In a further aspect, the present disclosure refers to a method for generating an aerosol stream, the method comprising the following steps:
(i) receiving a portion of breathing air from a ventilatory circuit to be provided to an aerosol generating element;
(ii) generating aerosol particles and introducing them into an aerosol stream; and
(iii) providing the aerosol stream comprising the aerosol particles through a conducting element to the patient.

In a particularly preferred embodiment, the aerosol stream comprising the aerosol particles may directly be provided to a patient interface. In a further preferred embodiment, an additional pressure may be provided within the aerosol stream for guiding the aerosol stream through the conducting element, which may, preferably, comprise at least one of a patient interface or an aerosol valve, to the patient.

For further details concerning the method for generating an aerosol stream, reference can be made to the aerosol generating device configured for generating an aerosol stream as described elsewhere herein.

In a further aspect, the present disclosure refers to a processing device, which is configured for controlling an apparatus for administering an aerosol to a patient by
- receiving input data related to a respiration pattern of a patient, wherein the respiration pattern comprises information about a temporal course of at least one of an elevation or a constriction of at least one of a chest or an abdomen of a patient;
- determining at least one point in time from the respiration pattern; and
- controlling an aerosol stream to the patient triggered at the at least one point in time,
wherein the at least one point in time advances at least one of an onset or a suspension of a breathing of the patient.

As used herein, the term "apparatus" refers to a device that comprises a plurality of elements, wherein each element cooperates with at least one further element to administer an aerosol to a patient. In particular, the apparatus can be embodied as a single device, which may comprise at least all elements required for this purpose. As an alternative, at least two elements of the apparatus may be placed on different locations, wherein each element may comprise or cooperate with a communication element configured for communication between the at least two elements of the apparatus. By way of example, the different locations may be in the same room, building, town, or country, or distributed over at least two continents. Further examples are conceivable.

As used herein, the term "processing" or any grammatical variation thereof refers to applying at least one algorithm to data received by at least one input file in a manner that the desired controlling of the apparatus for administering the aerosol to the patient is provided by at least one output file which comprises at least one command for controlling an aerosol valve configured for providing the aerosol stream to the patient. With particular regard to the present disclosure, the term "data" refers to at least one piece of information comprised by at least one file, specifically by at least one input file or at least one output file. With particular respect to the present disclosure, the at least one piece of information comprised by at least one input file is related to a respiration pattern of the patient, while the at least one piece of information comprised by at least one output file is related to the at least one command for controlling the aerosol stream to the patient which is triggered at at least one point in time. Herein, the at least one algorithm may be configured to determine the data for the at least one output file from the data provided by the at least one input file according to a predefined scheme, wherein, as described below in more detail, artificial intelligence, in particular at least one machine learning algorithm, may also be applied.

As generally used, the term "processing device" refers to a particular kind of device which is designated for determining the data for the at least one output file from the data provided by the at least one input file, wherein the at least one input file may, preferably, be provided to the processing device by using at least one input interface, and wherein the at least one output file may, preferably, be provided by the processing device by using at least one output interface. Specifically, the processing device may comprise at least one of an integrated circuit, in particular an application-specific integrated circuit (ASIC), or a digital processing device, in particular at least one of a digital signal processor (DSP), a field programmable gate array (FPGA), a microcontroller, a microcomputer, a computer, or a mobile communication device, specifically a notebook, a tablet, a smartphone, or a personal digital assistant. Further components may be feasible, in particular at least one of a preprocessing element or a data storage element. The processing device may, preferably, be designed to perform at least one computer program, in particular at least one line of computer program code configured to execute at least one algorithm for determining the data for the at least one output file, wherein the processing of the data may be performed in at least one of a consecutive or a parallel manner.

According to the present disclosure, the processing device is configured for controlling the apparatus for administering the aerosol to the patient, firstly, by receiving input data that are related to a respiration pattern of a patient. As generally used, the term "receiving" or any grammatical variation thereof refers to a process of obtaining at least one piece of information, in particular as at least one input file. With particular regard to the present disclosure, the at least one input file comprises data which are related to a respiration pattern of the patient. As further generally used, the term "respiration pattern" relates to a temporal course of an alteration of at least one body part of a patient owing to and related to a breathing of the patient. Herein, each respiration pattern can be identified by a first point in time which is related to an onset of the breathing of the patient and by a second point in time which is related to a suspension of the breathing of the patient, wherein the first point in time and the second point in time arise in an alternating manner, thereby defining a respiratory cycle. In many cases, the respiration pattern has a regular pattern being repeated after each respiratory cycle, wherein, typically, each respiratory cycle may have approximately the same time duration. However, alternatively or in addition, the respiration pattern may also comprise at least one irregular feature with respect to the respiration of the patient. Especially, a preterm infant or a seriously ill adult may, however, have a difference in the time duration for consecutive respiration patterns. Advantageously, the present technology a operates, as described below in more detail, independently of whether the respiration pattern may be a regular pattern or not.

Further, the processing device is configured for determining at least one point in time from the respiration pattern. As generally used, the term "determining" or any grammatical variation thereof refers to a process of generating representative results which are, typically, denoted as "data". With particular regard to the present disclosure, the data which are generated by the processing device corresponds to at least one output file comprising at least one piece of information that is related to at least one command, which is configured for controlling the aerosol stream to the patient that is triggered at the at least one point in time. For this purpose, the at least one command corresponds to at least one piece of information that is directed to altering a flow rate of the aerosol stream, in particular one of commencing, increasing, maintaining, decreasing, or terminating the volume of the aerosol stream. As described below in more detail, an aerosol valve configured for providing the aerosol stream to the patient can be controlled for this purpose, however, other procedures, such as altering the gas stream of the carrier gas, may, in principle, also be feasible.

Further, the processing device is configured for controlling the aerosol stream to the patient that is triggered at the at least one point in time. As generally used, the term "controlling" or any grammatical variation thereof refers to a process of adjusting at least one property of an object or a state by using at least one command. As further generally used, the term "triggering" or any grammatical variation thereof refers to a signal which is configured for commencing an alteration of the object or the state. With respect to the present disclosure, the aerosol stream to the patient is controlled in a manner that the flow rate of the aerosol stream is altered upon the signal being provided by the at least one command as determined by the processing device from the input data.

According to the present disclosure, the respiration pattern comprises information about the temporal course of at least one of an elevation or a constriction of at least one of a chest or an abdomen of a patient. As generally known, the chest and the abdomen of the patient experiences the elevation and the constriction in an alternating manner, in particular owing to the breathing of the patient, whereby the respiratory cycle is defined. As generally used, the term "elevation" refers to a rise of the abdominal wall and/or the rip cage, while the term "constriction" relates to a lowering of the abdominal wall and/or the rip cage, whereby they resume their preceding position. As indicated above, the respiratory cycle can be regular or not, wherein the present technology has a particular advantage in that it allows controlling the aerosol stream to the patient independently of whether the respiration pattern is regular or not.

Further, controlling the aerosol stream to the patient comprises a triggering of the aerosol stream at the at least one point in time, i.e. at at least one of the first point in time related to the onset of the breathing of the patient or the second point in time related to the suspension of the breathing of the patient, wherein a triggering of the aerosol stream at the first point in time may, generally, be preferred to the triggering of the aerosol stream at the second point in time. In particular accordance with the present disclosure, the at least one point in time advances at least one of the onset or the suspension of the breathing of the patient, wherein the onset and the suspension of the breathing of the patient may, in particular, correspond to the onset or the suspension of the aerosol stream through at least one of a nose or a mouth of the patient. As a consequence, while the first point in time is related to the onset of the breathing of the patient by being determined from the elevation of at least one of the chest or the abdomen of the patient, it, nevertheless, advances the onset of the breathing of the patient as indicated by an onset and a subsequent increase of a volume of the aerosol stream through at least one of the nose or the mouth of the patient. Similarly, while the second point in time is related to the suspension of the breathing of the patient by being determined from the constriction of at least one of the chest or the abdomen of the patient, it, nevertheless, advances the suspension of the breathing of the patient as indicated by a drop and a subsequent decrease of the volume of the aerosol stream through at least one of the nose or the mouth of the patient. In particular, the at least one point in time may advance the onset or the suspension of the breathing of the patient, respectively, by a time interval of 1 millisecond, preferably 50 milliseconds, more preferred of 100 ms, to 1 second, preferably 500 milliseconds, more preferred of 200 milliseconds; however, depending on the particular patient, a different value may also be feasible.

In other words, the aerosol stream to the patient may, preferably, be triggered by the at least one command as determined by the processing device from the input data which comprise information about the elevation or the constriction of at least one of the chest or the abdomen of the patient and, thereby, advances at least one of the onset or the suspension of the breathing of the patient in a favorable manner. It has been found that the input data which correspond to a movement of the chest or the abdomen of the patient are, particularly, suitable for determining the at least one point in time at which the triggering of the aerosol stream to the patient is released. While the movement of the nose and the mouth of the patient are synchronous with the breathing of the patient, the movement of the chest or the abdomen of the patient precede by the time interval as indicated above and can, therefore, be used for the purposes of the present disclosure, especially for administering a pharmaceutical preparation comprised by the aerosol to the patient, in particular to ensure that a predominant amount, preferably a complete amount, of the pharmaceutical preparation may actually be provided to the patient in a desired concentration and may not be diluted or distributed elsewhere.

This advantage is in particular contrast to the known prior art, according to which up to 90 vol.% or more of a pharmaceutical preparation may actually be distributed elsewhere apart from the patient, and according to which a, typically, high dilution of a pharmaceutical preparation during administering to the patient occurs. Rather, advancing the triggering of the aerosol by a small time interval with respect to the onset or the suspension of the breathing of the patient, respectively, results in a particular advantageous effect that the pharmaceutical preparation comprised by the aerosol is introduced into the respiratory gases at a point in time at which it can be ensured that the predominant amount, preferably the complete amount of the introduced pharmaceutical preparation may actually be received by the respiratory tracks of the patient, in particular at least one predefined target in at least one of a central or a peripheral area of the lung may be possible. Moreover, by near-patient breath-triggered release of the pharmaceutical preparation it may further be possible to reduce or, preferably, to avoid a delay in administering the pharmaceutical preparation to the patient. In this manner, an optimal breath-triggered release of the pharmaceutical preparation can be achieved, in particular by providing a cloud of aerosol to at least one of the nose or the mouth of the patient prior to the onset of inhalation flow. Such a kind of "pre-triggering" is provided by the present technology since it enables detecting an onset of the inhalation flow prior to measuring an onset of the respiratory flow. Similar results can also be achieved for the suspension of the respiratory flow by using the present technology. In addition, using the kind of input data according to the present technology, which have specifically been generated for this purpose, ensures that this effect is independent of whether the respiration pattern of the patient may be a regular pattern or not. As a result, the present technology can, especially, be employed for treating and curing preterm infants or seriously ill adults who, typically, exhibit an irregular respiration pattern

In a further aspect, the present invention refers to an apparatus for administering an aerosol to a patient, according to claim 12.

According to some embodiments, the apparatus for administering the aerosol to the patient comprises an aerosol valve which is configured for providing the aerosol stream to the patient and, in addition, the aerosol generating device and the processing device as described above and below in more detail, wherein the aerosol generating device comprises an aerosol generating element being configured for generating aerosol particles and introducing them into the aerosol stream, wherein the processing device is configured for controlling the aerosol stream through the aerosol valve by triggering the aerosol valve at the at least one point in time, whereby the aerosol stream designated for being provided to the patient is adjusted in the manner as disclosed herein. As an alternative, the apparatus according to the present disclosure may comprise the aerosol valve and the aerosol generating device as disclosed herein, and a prior art processing device, or, as a further alternative, the aerosol valve and the processing device as disclosed herein, and a prior art aerosol generating device.

As generally used, the term "aerosol valve" refers to a device which is configured for controlling a volume of an aerosol stream, especially for being provided to at least one of the nose or the mouth of the patient. As indicated above, the aerosol valve can be controlled by at least one command comprising at least one piece of information which is directed to altering a flow rate of the aerosol stream, in particular one of commencing, increasing, maintaining, decreasing, or terminating the volume of the aerosol stream. For this purpose, a pneumatic device which can be addressed by the at least one command may be used; however, a further embodiment may also be feasible. In a preferred embodiment, the aerosol valve can be integrated into the patient interface which is, as described above, configured for providing a connection between the ventilatory circuit and the respiratory track of the patient. As an alternative, the aerosol valve can be located in an upstream position with respect to the patient interface. Further alternatives are conceivable.

In a preferred embodiment, the apparatus for administering the aerosol to the patient may, further, comprise a respiratory detection device. As used herein, the term "respiratory detection device" is a device which is configured for determining the input data related to the respiration pattern of the patient and for transmitting the input data related to the respiration pattern of the patient to the processing device as disclosed herein.

The respiratory detection device may be configured for determining at least one of the elevation or the constriction of at least one of the chest or the abdomen of the patient, which are related to the respiration of the patient. In a particular embodiment, the respiratory detection device may at least comprise
- a detection element configured for recording at least one of the elevation or the constriction of at least one of the chest or the abdomen of the patient;
- a processing element configured for determining the input data related to the respiration pattern of the patient from at least one of the elevation or the constriction of the at least one of the chest or the abdomen of the patient; and
- a communication interface configured for transmitting the input data to the processing device.

Additional components are conceivable.

As used herein, the term "detection element" refers to device or a portion thereof, which is configured for recording and measuring at least one physical property related to a movement of a body part. Herein, the detection element may operate in contact with the body of the patient or, alternatively or in addition, in a contactless manner. In particular, the detection element may, be a breath detection sensor selected from at least one of a strain-gauge element, a time-of-flight camera, an electrical impedance tomography sensor, a respiratory inductive plethysmography sensor, a millimeter wave sensor, a radar sensor, or a thermal sensor. However, using a different type of detection element may also be feasible.

As further used herein, the term "processing element" refers to a further device or a portion thereof, which is configured for determining at least one piece of data. Herein, the processing element and the communication interface may, preferably, constitute a single device, while the detection element may constitute a separate device. An embodiment in which the processing element, the detection element and the communication interface may form an integrated device may also be feasible. The processing element may, in particular, be configured for receiving measurement data about at least one of the elevation or the constriction of at least one of the chest or the abdomen of the patient from the detection element, wherefrom it may determine the input data related to the respiration pattern of the patient.

As further used herein, the term "communication interface" refers to a transmission channel which is designated for transmitting data from a first location to a second location, which, particularly, differs from the first location. Preferably, the communication interface may be a unidirectional interface configured for forwarding data into a single direction, such as from the processing element to the processing device. Alternatively, the communication interface may be a bidirectional interface configured for forwarding data into one of two directions, such as from the processing element to the processing device, or vice versa, in particular for further transmitting at least one command from the processing device to the processing element, wherein the at least one command may be selected from commencing or finishing a measurement, or commencing or finishing a data transmission. For a purpose of data transmission, the communication interface may comprise at least one of a wire-bound element or a wireless element, wherein the wireless element may be configured to operate by using a wireless communication protocol, such as Wi-Fi or Bluetooth; however, a further kind of communication interface may also be feasible. In a particular embodiment, the communication may be or comprise an encrypted data transfer or an encrypted data exchange, especially for a protection of personal data.

For details concerning the apparatus for administering the aerosol to the patient, reference can be made to the description of the processing device and of the exemplary embodiments elsewhere in this document.

In a further aspect (not being object of the present invention) a method for administering an aerosol to a patient is proposed, wherein the method comprises the following steps a) to c):
a) receiving input data related to a respiration pattern of a patient, wherein the respiration pattern comprises information about a temporal course of at least one of an elevation or a constriction of at least one of a chest or an abdomen of a patient;
b) determining at least one point in time from the respiration pattern; and
c) controlling an aerosol stream to the patient triggered at the at least one point in time,
wherein the at least one point in time advances at least one of an onset or a suspension of a breathing of the patient.

Herein, the indicated steps a) to c) may be performed in the order as provided, wherein, preferably, all of the indicated steps may be preformed at least partially concurrently. Further, additional method steps, whether described in this document or not, can be performed, too.

The method for administering an aerosol to a patient as disclosed herein may, preferably, be a computer-implemented method. As generally used, the term "computer-implemented method" refers to a method involving at least one programmable device, particularly, selected from a mobile communication device. However, a further kind of programmable device, such as may also be feasible. Herein, the at least one programmable device may, especially, comprise the processing device as disclosed herein or have access thereto, wherein at least one feature of the method is performed by using at least one computer program. For this purpose, the computer program may be provided on the at least one programmable device, or the at least one programmable device may have access to the computer program via a network, such as an in-house network or the internet, which may be located in a remote server or a cloud.

According to step a), the input data which are related to the respiration pattern of the patient are received, wherein the respiration pattern comprises information about the temporal course of at least one of the elevation or the constriction of at least one of the chest or the abdomen of the patient.

According to step b), at least one point in time is determined from the respiration pattern as described elsewhere herein in more detail.

According to step c), the aerosol stream to the patient is controlled by triggering the aerosol stream at the at least one point in time, wherein the at least one point in time advances the at least one of the onset or the suspension of the breathing of the patient.

For details concerning the method for administering the aerosol to the patient, reference can be made to the description of the processing device, the apparatus for administering an aerosol to a patient, and the exemplary embodiments elsewhere in this document.

The apparatus, devices and methods according to the present disclosure offer various advantages with respect to known apparatus, devices and methods. The processing device and the corresponding method are, particularly, configured to use respiratory motion signals comprised by the respiration patterns in their mostly raw, minimally analyzed form. This advantage eliminates a need for a predictive analysis. In particular contrast to the disclosure of US 10 987 474 B2, WO 2020/243107 A1 and US 2020/368457 A1, no prior analysis of respiration pattern is required for predicting breaths of the patient. Therefore, no reliance on predictive algorithms is required. The processing device and the corresponding method do not use any algorithms for pattern recognition and/or for predicting respiratory events over a temporal course of the breathing. Rather, real-time signals related to movements, i.e. an elevation or a constriction, of the chest and/or the abdomen of the patient are used for indicating the onset or suspension of the breathing of the patient. As advantages thereof, an immediate and responsive aerosol delivery is obtained. The aerosol stream can respond without delay to real-time changes in the breathing cycle. Further, this arrangement exhibits a reduced complexity, particularly since no sophisticated pattern recognition or predictive algorithms are needed. These advantages provide an increased accuracy and efficiency with regard to drug delivery in natural breathing cycles as well as in irregular respiration patterns. The aerosol stream can be synchronized precisely with each breath, making it suitable for patients with irregular respiration patterns. This results in a precise delivery of the aerosol stream to the patient, in particular by ensuring accurate medication delivery at the start of the inhalation, by maximizing efficacy and by minimizing waste. In contrast hereto, known prediction models, such as disclosed in US 10 987 474 B2, WO 2020/243107 A1 and US 2020/368457 A1, are prone to errors when applied to irregular breathing, whereas the present technology overcomes these disadvantages by using a direct measurement approach. In further contrast to using a prior art mesh system that requires a lead time to aerosolize the drug, the aerosol generating device according to the present disclosure can continuously produce and transport the aerosol stream to the aerosol valve, wherein the aerosol valve can directly open at the onset of the breathing so that the aerosol stream is immediately available to the patient. Similarly, the aerosol valve can directly close at the suspension of the breathing so that the aerosol stream is immediately terminated, thereby reducing waste.

As a further advantage of the apparatus, devices and methods according to the present disclosure, different areas of the lungs can be reached via the time of release. If the aerosol may be administered to the patient directly at the start of inhalation, the peripheral regions of the lungs can primarily be treated. If the aerosol may be administered towards the end of the inhalation, the central regions of the lungs can primarily be treated. In addition, bolus delivery could be used to specifically treat well-ventilated or poorly ventilated areas of the lungs, as is the case with pulsed delivery of medical gases. Time-efficient and targeted inhalation therapy is, therefore, possible, which can contribute to a reduction in the dose of the administered medication and, thus, to fewer side effects.

As used herein, the terms "have", "comprise" or "include" or any arbitrary grammatical variations thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The present invention is defined by the appended claims.

### Short description of the Figures

Further optional features and embodiments of the technology will be disclosed in more detail in the subsequent description of preferred embodiments. Therein, the respective optional features may be implemented in an isolated fashion as well as in any arbitrary feasible combination, as the skilled person will realize. It is emphasized that the scope of the invention may not be restricted by the preferred embodiments. The embodiments are schematically depicted in the Figures. Therein, identical reference numbers in these Figures refer to identical or functionally comparable elements.

In the Figures:
- Figure 1: schematically illustrates a preferred embodiment of an exemplary apparatus for administering an aerosol to a patient according to the present technology;
- Figure 2: schematically illustrates a comparison between temporal courses of first signals generated by using a strain-gauge element and of second signals generated by using a flow sensor during an exhalation phase (Fig. 2A) and an inhalation phase (Fig. 2 B), respectively;
- Figure 3: schematically illustrates schematically illustrates respiratory phases of a preterm infant extracted from an abdominal motion data recorded with a time-of-flight camera; and
- Figure 4: schematically illustrates a comparison of a dose efficiency of a pharmaceutical preparation for various tests with respect to an emitted dose.

### Detailed description of the embodiments

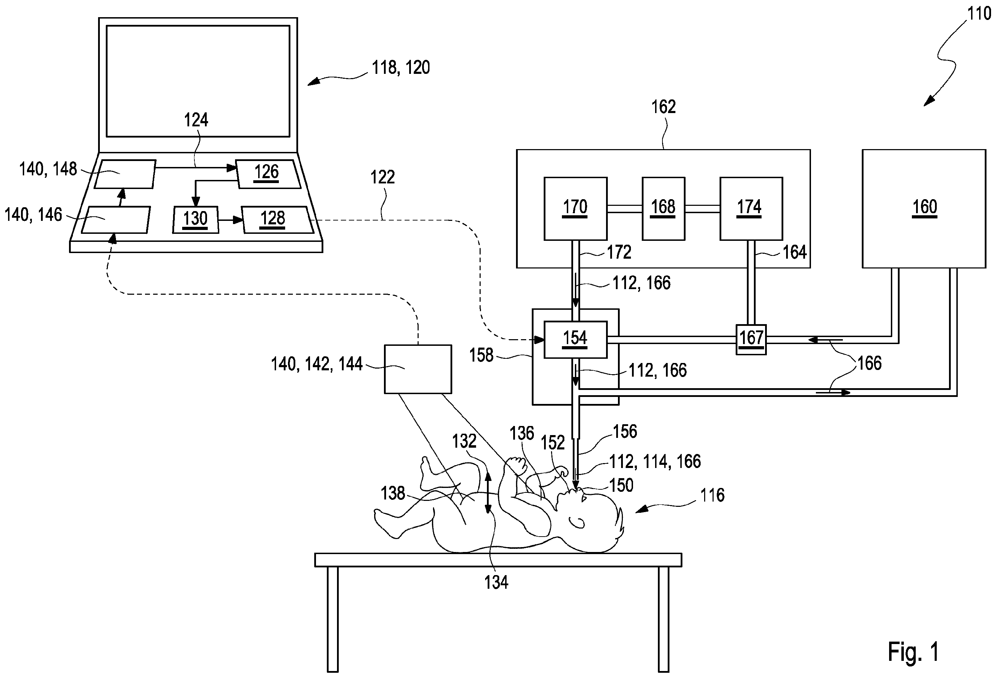

Figure 1 schematically illustrates a preferred embodiment of an exemplary apparatus 110 for administering an aerosol 112, in particular in form of an aerosol stream 114, to a patient 116. It is emphasized here that the exemplary embodiment of the apparatus 110 as schematically illustrated in Figure 1 comprises both a processing device 120 and an aerosol generating device 162, however, it is also feasible to use only the processing device 120 or the aerosol generating device 162. In particular, the apparatus 110 is designed for providing the aerosol stream 114 having a defined aerosol concentration, preferably a high aerosol concentration, to the patient 116, who may, especially, be a preterm infant, a newborn, a child, or a seriously ill adult. However, further applications of the apparatus 110 are feasible.

Further, the exemplary apparatus 110 as depicted in Figure 1 comprises a notebook 118, which is or comprises the processing device 120 configured for controlling the apparatus 110 for administering the aerosol 112, preferably in form of the aerosol stream 114, to the patient 116. As an alternative, the processing device 120 may be or be comprised by a different kind of mobile communication device, especially a tablet, a smartphone, or a personal digital assistant. As a further alternative, at least one of an integrated circuit, in particular an application-specific integrated circuit (ASIC), or a digital processing device, in particular at least one of a digital signal processor (DSP), a field programmable gate array (FPGA), a microcontroller, a microcomputer, or a computer can be used as the processing device 120.

The processing device 120 is designated for determining data for at least one output file 122 from data provided by the at least one input file 124, wherein the at least one input file 124 may be provided to the processing device 120 by using an input interface 126, and wherein the at least one output file 122 may be provided by the processing device 120 by using an output interface 128. The processing device 120 as shown in Figure 1 is further designed to perform at least one computer program, in particular at least one line of computer program code, configured to execute at least one algorithm 130 for determining the data for the at least one output file 122, wherein the processing of the data can be performed by using the at least one algorithm 130 in a consecutive and/or a parallel manner.

For this purpose, the processing device 120 is configured for receiving the at least one input file 124 comprising input data related to a respiration pattern of the patient 116. The respiration pattern in this exemplary embodiment comprises information about a temporal course of an elevation 132 and a constriction 134 of a chest 136 or an abdomen 138 of the patient 116. In this manner, information about a rise and a lowering of an abdominal wall and a rip cage of the patient 116 can be obtained, wherefrom the respiration pattern can be derived. In general, the respiration pattern may be a regular respiration pattern or, especially for a preterm infant or a seriously ill adult, an irregular respiration pattern, wherein the present technology has a particular advantage in that it allows controlling the aerosol stream 114 to the patient 116 independently of whether the respiration pattern may be regular or not.

The exemplary apparatus 110 as depicted in Figure 1, further, comprises a respiratory detection device 140, which is configured for determining the at least one input file 124 comprising the input data related to the respiration pattern of the patient 116 and for transmitting the at least one input file 124 to the processing device 120 by using the input interface 126. With particular respect to the present technology, the respiratory detection device 140 is configured in this exemplary embodiment for determining both the elevation 132 and the constriction 134 of the chest 136 and/or the abdomen 138 of the patient 116, which are related to the respiration of the patient 116.

As further illustrated in Figure 1, the respiratory detection device 140 comprises a detection element 142, which is configured in this exemplary embodiment for recording both the elevation 132 and the constriction 134 of the chest 136 and/or the abdomen 138 of the patient 116. In general, the detection element 142 may operate in contact with the body of the patient 116 or, alternatively or in addition, in a contactless manner. As depicted there, the detection element 142 is or comprises a time-of-flight camera 144, which is configured for recording and measuring at least one physical property related to a movement of the chest 136 and/or the abdomen 138 of the patient 116 in a contactless manner. As an alternative, a different type of a breath detection sensor can be used as the detection element 142, especially a contact-providing element, such as a strain-gauge element, or a different contactless element, such as an electrical impedance tomography sensor, a respiratory inductive plethysmography sensor, a millimeter wave sensor, a radar sensor, or a thermal sensor.

As further illustrated in Figure 1, the respiratory detection device 140 further comprises the notebook 118 which, additionally, has a processing element 146, which is configured for determining the at least one input file 124 comprising the input data related to the respiration pattern of the patient 116, and a communication interface 148, which is configured for transmitting the at least one input file 124 comprising the input data related to the respiration pattern of the patient 116 to the input interface 126 comprised by processing device 120.. In the exemplary embodiment of Figure 1, the processing element 146 and the communication interface 148 are both comprised by the notebook 118, while the detection element 142 is embodied as a separate device. However, other embodiments are feasible, in particular an embodiment in which the detection element 142, the processing element 146 and the communication interface 148 may form an integrated device (not depicted here). For a purpose of data transmission, the communication interface 148 may comprise at least one of a wire-bound element or a wireless element configured to operate by using a wireless communication protocol, such as Wi-Fi or Bluetooth, wherein an encrypted data transfer or an encrypted data exchange, especially for a protection of personal data, may also be feasible.

The communication interface 148 may be or comprise a unidirectional interface configured for transmitting data into a single direction from the processing element 146 to the input interface 126 comprised by processing device 120. Alternatively, the communication interface 148 may be a bidirectional interface configured for forwarding data into one of two directions, such as from the from the processing element 146 to the input interface 126, or vice versa, in particular for further transmitting at least one command from the processing device 120 to the processing element 146, wherein the at least one command may be directed to commencing or finishing a measurement, or commencing or finishing a data transmission.

Further, the processing device 120 is configured for determining at least one point in time from the respiration pattern and for controlling the aerosol stream 114 to the patient 116 triggered at the at least one point in time, wherein the at least one point in time advances an onset and/or a suspension, respectively, of a breathing of the patient. In particular, the at least one point in time may advance the onset or the suspension, respectively, of the aerosol stream 114 through a nose 150 or a mouth 152 of the patient 116. More particular, the at least one point in time may advance the onset or the suspension, respectively, of the breathing of the patient 116 by a time interval of 1 millisecond, preferably 50 milliseconds, more preferred of 100 ms, to 1 second, preferably 500 milliseconds, more preferred of 200 milliseconds; however, depending on the particular patient 116, a different value may also be feasible. In this respect, the aerosol stream 114 to the patient 116 is triggered by at least one command as comprised by the at least one output file 122 as determined by the processing device 120 by using the at least one algorithm 130, wherein the at least one output file 122 comprises at least one command being forwarded by using an output interface 128 to an aerosol valve 154 being configured for providing the aerosol stream 114 to the patient 116.

It has been found that using the at least one input file 124 comprising the input data related to the respiration pattern of the patient 116 as determined by using the information about the elevation 132 and/or the constriction 134 of the chest 136 and/or the abdomen 138 of the patient 116 allows using the processing device 120 for predicting the at least one point in time at which the triggering of the aerosol stream 114 to the patient 116 can reasonably be released. In contrast to the flow movement through the nose 150 and the mouth 152 of the patient 116, which are synchronous with the breathing of the patient 116, the movement of the chest 136 and/or the abdomen 138 of the patient 116 precede by the time interval as indicated above, which can, therefore, be used for the purposes of the present technology, especially for administering a pharmaceutical preparation comprised by the aerosol 112 to the patient 116. In this manner, it can be ensured that a predominant amount, preferably a complete amount, of the pharmaceutical preparation may actually be provided to the patient 116 and not distributed elsewhere, such as to an undesired portion of the apparatus 110 or to surroundings of the apparatus 110. By using the at least one input file 124 it can, further be ensured that this effect is independent of whether the respiration pattern of the patient may be regular or not.

In further accordance with the present technology, the apparatus 110 further comprises the aerosol valve 154, which is configured for providing the aerosol stream 114 to the patient 116. For this purpose, the aerosol valve 154 is configured for controlling a volume of the aerosol stream 114 which is, particularly, provided to the nose 150, such as by using a pair of nasal prongs 156, or to the mouth 152 of the patient 116. As indicated above, the aerosol valve 154 can be controlled by the at least one command comprising at least one piece of information being directed to altering a flow rate of the aerosol stream 114, in particular one of commencing, increasing, maintaining, decreasing, or terminating the volume of the aerosol stream 114. In the exemplary embodiment of Figure 1, the aerosol valve 154 is integrated into a patient interface 158 configured for providing a connection between a ventilatory circuit 160 and a respiratory track of the patient. As an alternative (not depicted here), the aerosol valve 154 can be located at a different position, such as upstream with respect to the patient interface 158.

As further illustrated Figure 1, the exemplary embodiment of the apparatus 110 comprises the aerosol generating device 162, which is configured for generating the aerosol stream 114 to be provided to the patient 116, particularly preferred directly to the patient interface 158. In particular, the aerosol generating device 162 as depicted there comprise an air inlet 164 configured for receiving a portion of breathing air 166 via a junction 167 from the ventilatory circuit 160, which is provided here to a breathing filter 168 configured for removing at least one interfering substance from the breathing air 166 before being providing to an aerosol generating element 170 as further comprised by the aerosol generating device 162. Herein, the aerosol generating element 170 may, especially, be configured for generating aerosol particles and introducing them into the aerosol stream 114. For this purpose, the aerosol generating element 170 may be designated for converting an aerosolizable material, i.e. a powder or a liquid solution, into the desired aerosol 112, in particular by using a powder generator or a nebulizer, especially vibrating meshes or ultrasonic waves, in order to entrain the solid or liquid particles into the gas stream that may comprise the breathing air 166 and a carrier gas, such as a respiratory gas. Herein, the aerosol generating element 170 may, preferably, be selected from a nebulizer and/or a powder generator. As further depicted in Figure 1, the aerosol generating device 162 further comprises an aerosol tube 172 which is configured for providing the aerosol 112 comprising the aerosol particles through a conducting element, in particular the aerosol valve 154 and/or the patient interface 158, to the patient 116. However, using a tube (not depicted here) may also be feasible.

According to the present invention, as illustrated in Figure 1, the aerosol generating device 162, further, comprises a pressure modulation element 174, which is configured for providing an additional pressure within the aerosol stream 114. In particular, the additional pressure may be used for improving a manner of guiding of the aerosol stream 114 to the patient 116 through the conducting element, in particular the aerosol valve 154 and/or the patient interface 158. According to the present invention, a pressure prevailing at the aerosol tube 172, when the aerosol 112 is administered to the patient 116, in particular when the aerosol valve 154 is in an open position, exceeds a further pressure prevailing at the air inlet 164 by at least 0.1 mbar, preferably by at least 0.05 mbar, more preferred by at least 0.02 mbar, especially by at least 0.01 mbar. Preferably, the pressure modulation element 174 may, further, be configured for limiting the additional pressure within the aerosol generating device to an additional peak pressure, wherein, the additional peak pressure may assume a value of at maximum 20 mbar or 10 mbar, preferably 5 mbar, more preferred 2 mbar, especially 1 mbar, or below. Limiting the additional peak pressure in the patient interface 158, when the aerosol 112 is administered to the patient 116, in particular when the aerosol valve 154 is in an open position, can contribute to avoid damaging the patient 116 by a too high pressure. In this manner, a volume flow of 0.01 *L*/*min* to 3 *L*/*min,* on average approx. *1 L*/*min,* can, preferably, be generated by the pressure modulation element 174. Herein the low volume flow of 0.01 *L*/*min* to 3 *L*/*min,* on average approx. *1 L*/*min,* compared to the volume flow in the ventilatory circuit 160, enables a high, virtually undiluted aerosol concentration to be transported directly to the patient 116.

If the aerosol stream 114 is injected - according to the prior art - directly into the ventilatory circuit 160, this would lead to a strong dilution of the aerosol concentration. By way of example, using a breathing gas flow of 7 *L*/*min,* an aerosol output of 4.5 *mg*/*min* from the prior art aerosol generator and a homogeneous distribution of the aerosol particles, would achieve an aerosol concentration of 0.75 *mg*/*L* in the breathing gas. In contrast hereto, by using a small partial flow removed from the breathing gas upstream of the patient interface 158, enriched with the aerosol and fed directly into the patient interface 158 according to the present technology the aforementioned dilution effect can be avoided. If the aerosol transport flow is reduced, the aerosol concentration increases. By way of example, the aerosol may be transported to the patient interface 158 by using a low transport flow of only *1 L*/*min,* independent of the respiratory gas flow in the ventilation system, which may lead to an aerosol concentration of 4.5 *mg*/*L* in the in breathing gas, thus being 6 times higher compared to the prior art. As a result, a dose inhaled per time unit by a patient can be considerably increased. By additionally using a breath-synchronized drug delivery device, as described above, an efficiency of aerosol deposition in the lungs can further be increased.

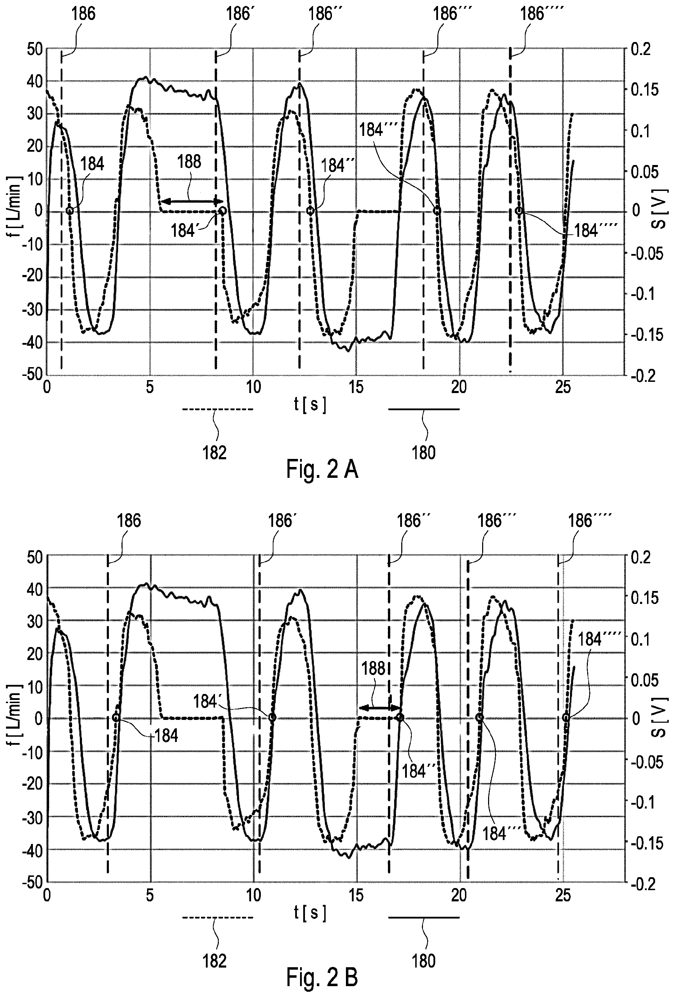

Figure 2 schematically illustrates a comparison between a first temporal course 180 of first signals generated by using a strain-gauge element and a second temporal course 182 of second signals generated by using a flow sensor during an exhalation phase (Fig. 2A) and an inhalation phase (Fig. 2B), respectively. Herein, the x-axis shows a time t in seconds s, the left y-axis a respiratory flow *f* as measured by using the flow sensor in *L*/*min,* and the right y-axis a mean signal S generated by the strain-gauge element in *volts.* A respective switch from exhalation to inhalation and vice versa is indicated by circles 184, 184', 184", ... for the flow sensor and by vertical dashed lines 186, 186', 186", ... for the strain-gauge element. As indicated by a horizontal line 188, breathing was stopped twice in order to be able to accurately represent the onset of the inhalation and of the exhalation, respectively. A change from inhalation to exhalation is indicated by a zero crossing of the y-axis for the flow sensor and by extreme values or an abrupt slope increase or slope decrease, respectively, for the strain-gauge element. As shown in Figure 2, a maximum inhalation is followed by a maximum abdominal stretch, resulting in a maximum stretch of the strain-gauge element and, thus, a maximum positive tension. Similarly, a maximum exhalation is followed by minimum abdominal stretch, resulting in a minimum stretch of the strain-gauge element and, thus, a maximum negative stress. As shown in Figure 2, the strain-gauge element is capable of detecting the inhalation or the exhalation, respectively, for a time interval of about 200 ms to 300 ms earlier than the flow sensor. This time interval, by which the strain-gauge element is capable of detecting the oncoming inhalation or the exhalation earlier compared to the flow sensor, allows advancing the triggering of the aerosol 112 with respect to the onset or the suspension of the breathing of the patient 116, respectively, thus resulting in the advantageous effects described elsewhere herein.

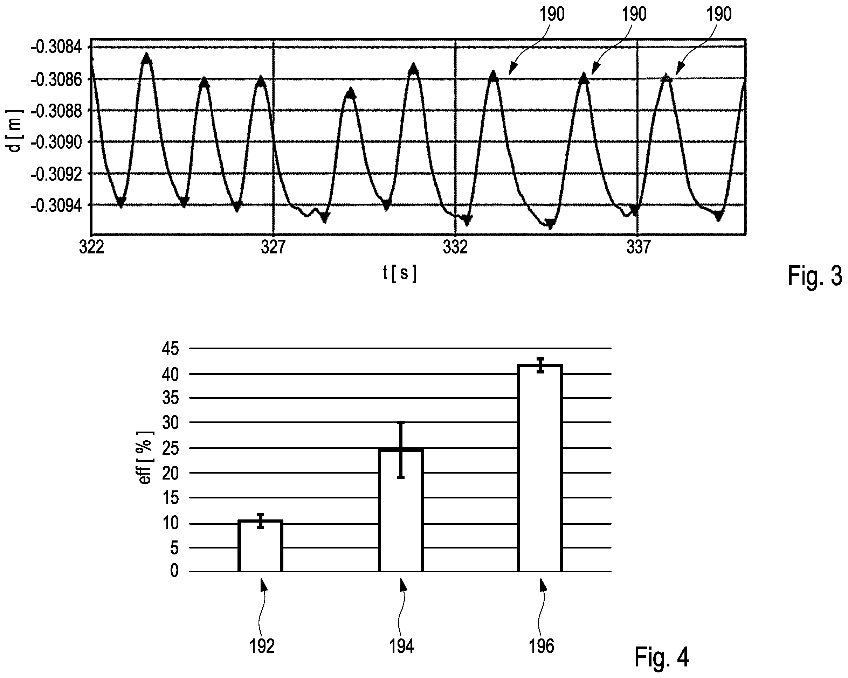

Figure 3 schematically illustrates respiratory phases 190 of a preterm infant extracted from an abdominal motion data recorded with a time-of-flight camera 144. Herein, a distance *d* in meters *m* from the time-of-flight camera 144 to the preterm infant's abdomen 138 is plotted versus time t in seconds s. The maxima and minima as depicted in Figure 3 represent transitions from inhalation to exhalation and vice versa.

Figure 4 schematically illustrates a comparison of a dose efficiency *eff* of a pharmaceutical preparation for various tests 192, 194, 196 in % with respect to an emitted dose. In a first test 192, a standard patient interface according to the prior art was used, in a second test 194 the aerosol generating device 162 in a bypass arrangement without using the aerosol valve 154 as disclosed herein was used, while and in a third test 196 the apparatus 110 according to the present technology including the aerosol generating device 162 by using the aerosol valve 154 as disclosed herein was used.

For this purpose, a 0.9 vol.% solution of saline was aerosolized using a mesh nebulizer and delivered to a respective test stand according to the above-mentioned tests 192, 194, 196 as follows:
- when using the using a standard patient interface in the first test 192, the aerosol was coupled into the ventilatory circuit 160 by using a Y-connector;
- when using the aerosol generating device 162 in a bypass arrangement without using the aerosol valve 154 in the second test 194, the aerosol 112 was delivered directly into the patient interface 158 at a flow of approx. *1 L*/*min.* The ventilatory circuit 160 was operated in continuous positive airway pressure (CPAP) mode using a positive end-expiratory pressure (PEEP) of 5 mbar and a respiratory gas flow of 6 *L*/*min.* For determining the delivered amount of aerosol 112, an advanced test rig as disclosed in WO 2020/007858 A1 was used. This test rig allows using different respiratory rates (30-80 breaths/min), tidal volumes (2-40 ml) and inhalation fractions (0.25-0.75). For the second test 194, a frequency of 51 breaths/min, a tidal volume of 12.3 ml, and an inspiratory fraction of 0.39 were used as simulated respiratory parameters; and
- when using the apparatus 110 according to the present technology in the third test 196, the same conditions were applied as in the second test 194, however, the aerosol valve 154 was used and was opened in the triggered mode of operation at the beginning of a simulated inspiration and closed at the beginning of a simulated expiration. For this purpose, the aerosol valve 154 was closed or opened by using a pneumatic device.

According to the results as depicted in Figure 4, the dose efficiency *eff* achieved in the first test 192 assumed a value of 10.3%, in the second test 194 of 24.7% (non-triggered release) and in the third test 196 of 41.5% (triggered release), respectively. Consequently, applying the non-triggered release by using the aerosol generating device 162 is more efficient than using the standard patient interface by a factor of approx. 2.5. Further, using the breath-triggered release in combination with the aerosol generating device 162 is more efficient by a factor of approx. 1.7 compared to the non-triggered release and by a factor of approx. 4.2 with respect to using the standard patient interface.

Further, aerosol measurements were performed under realistic clinical conditions in order to compare the aerosol generating device 162 according to the present technology with a prior art standard inhalative clinical (SoC) system. A test stand based on the disclosure of WO 2020/007858 A1 was used for simulating respiratory parameters of preterm infants. This test stand allows using different respiratory rates (30-80 *breaths*/*min),* tidal volumes (2-40 *ml)* and inspiratory fractions (0.25-0.75). A respiratory rate of 50 *breaths*/*min,* a tidal volume of *8 ml* and an inspiration to expiration ratio of 1:1.5 were used as respiratory parameters. A Babylog^{®} 8000 plus produced by Drägerwerk AG und Co. KGaA, Lübeck, Germany, was used to operate the ventilatory circuit 160 in a continuous positive airway pressure mode, applying a positive end-expiratory pressure of 5 *mbar* and a respiratory gas flow of 6 *L*/*min. 3 ml* of a solution comprising equal parts budesonide (0.125 *mg*/*ml)* and saline (0.9 %) was nebulized by using a mesh nebulizer produced by Aerogen Solo, Aerogen Ltd., Galway, Ireland.

The aerosol was delivered to the test stand
- either via a prior art SoC system produced by FlexitrunkTM, Fisher & Paykel Healthcare Limited, Panmure, Auckland, New Zealand, wherein the aerosol was delivered directly into the ventilatory circuit; or
- via the aerosol generating device 162 according to the present technology, wherein the aerosol was delivered directly into the patient interface 158 at a flow rate of approximately 1 *L*/*min.*

As experimentally demonstrated, a measured inhaled dose obtained by using the prior art SoC system was 3.0 % compared to 9.7 % as obtained by using the aerosol generating device 162 according to the present technology. As a consequence thereof, using the aerosol generating device 162 according to the present technology, advantageously, allows achieving a considerable increase in inhalation efficiency by a factor of 3.2 compared to the prior art SoC system.

### List of reference numbers

- 110: apparatus
- 112: aerosol
- 114: aerosol stream
- 116: patient
- 118: notebook
- 120: processing device
- 122: output file
- 124: input file
- 126: input interface
- 128: output interface
- 130: algorithm
- 132: elevation
- 134: constriction
- 136: chest
- 138: abdomen
- 140: respiratory detection device
- 142: detection element
- 144: time-of-flight camera
- 146: processing element
- 148: communication interface
- 150: nose
- 152: mouth
- 154: aerosol valve
- 156: nasal prong
- 158: patient interface
- 160: ventilatory circuit
- 162: aerosol generating device
- 164: air inlet
- 166: breathing air
- 167: junction
- 168: breathing filter
- 170: aerosol generating element
- 172: aerosol tube
- 174: pressure modulation element
- 180: first temporal course
- 182: second temporal course
- 184, 184', ....: circle
- 186, 186", ...: vertical dashed line
- 188: horizontal line
- 190: respiratory phase
- 192: first test
- 194: second test
- 196: third test

## Claims

1. An aerosol generating device (162) configured for generating an aerosol stream (114), comprising:
- an air inlet (164) configured for receiving a portion of breathing air (166) from a ventilatory circuit (160) to be provided to an aerosol generating element (170);
- the aerosol generating element (170) configured for generating aerosol particles and introducing them into the aerosol stream (114);
- an aerosol tube (172) configured for providing the aerosol stream (114) comprising the aerosol particles through a conducting element to the patient (116); and
- a pressure modulation element (174),
wherein the pressure modulation element (174) is configured for providing an additional pressure within the aerosol stream (114) for guiding the aerosol stream (114) through the conducting element to the patient (116),
**characterized in that**,
when the aerosol (112) is administered to the patient (116), a pressure prevailing at the aerosol tube (172) exceeds a further pressure prevailing at the air inlet (164) by at least 0.01 mbar.

2. The aerosol generating device (162) according to the preceding claim, wherein the aerosol generating element (170) is selected from at least one of a nebulizer or a powder generator.

3. The aerosol generating device (162) according to any one of the preceding claims, wherein the conducting element comprises at least one of an aerosol valve (154) or a patient interface (158).

4. The aerosol generating device (162) according to any one of the preceding claims, wherein the air inlet (164) is configured for receiving the portion of breathing air via a junction (167) from the ventilatory circuit (160).

5. The aerosol generating device (162) according to any one of the preceding claims, wherein the additional pressure is configured for improving a manner of guiding of the aerosol stream (114) to the patient (116) through the conducting element.

6. The aerosol generating device (162) according to any one of the preceding claims, wherein, when the aerosol (112) is administered to the patient (116), a pressure prevailing at the aerosol tube (172) exceeds a further pressure prevailing at the air inlet (164) by at least 0.1 mbar.

7. The aerosol generating device (162) according to any one of the preceding claims, wherein the pressure modulation element (174) is selected from at least one of a ventilator, a fan, a pump, a mechanical element or an electromechanical element configured for this purpose.

8. The aerosol generating device (162) according to any one of the preceding claims, wherein the pressure modulation element (174) is further configured for limiting the additional pressure within the aerosol generating device (162) to an additional peak pressure.

9. The aerosol generating device (162) according to the preceding claim, wherein the additional peak pressure assumes a value of at maximum 20 mbar or below.

10. The aerosol generating device (162) according to any one of the two preceding claims, wherein the pressure modulation element (174) is configured to generate a volume flow of 0.01 *L*/*min* to 3 *L*/*min,* which, compared to the volume flow in a ventilation circuit, is configured to directly transport an undiluted aerosol concentration to the patient (116).

11. The aerosol generating device (162) according to any one of the preceding claims, wherein the aerosol generating device (162) further comprises a breathing filter (168) upstream of the aerosol generating element (170), wherein the breathing filter (168) is configured for removing at least one interfering substance from the breathing air (166) to be provided to the aerosol generating element (170).

12. An apparatus (110) for administering an aerosol (112) to a patient (116), comprising:
- an aerosol valve (154) configured for providing an aerosol stream (114) to the patient (116); and
- an aerosol generating device (162) configured for generating the aerosol stream (114) according to any one of the preceding claims directed to the aerosol generating device (162).

13. The apparatus (110) according to the preceding claim, further comprising a respiratory detection device (140), wherein the respiratory detection device (140) is configured for determining at least one of the elevation (132) or the constriction (134) of the at least one of the chest (136) or the abdomen (138) of the patient (116) related to the respiration of the patient (116).

14. The apparatus (110) according to the preceding claim, wherein the respiratory detection device (140) comprises
- a detection element (142) configured for recording at least one of the elevation (132) or the constriction (134) of the at least one of the chest (136) or the abdomen (138) of the patient (116);
- a processing element (146) configured for determining the input data related to the respiration pattern of the patient (116) from the at least one of the elevation (132) or the constriction (134) of the at least one of the chest (136) or the abdomen (138) of the patient (116); and
- a communication interface (148) configured for transmitting the input data to the processing device (120).

15. The apparatus (110) according to the preceding claim, wherein the detection element (142) is a breath detection sensor selected from at least one of a time-of-flight camera (144), a strain-gauge element, an electrical impedance tomography sensor, a respiratory inductive plethysmography sensor, a millimeter wave sensor, a radar sensor, or a thermal sensor.

## Patentansprüche

1. Aerosolerzeugungsvorrichtung (162), die zum Erzeugen eines Aerosolstroms (114) eingerichtet ist, umfassend:
- einen Lufteinlass (164), der zum Aufnehmen eines Teils von Atemluft (166) aus einem Beatmungskreislauf (160) eingerichtet ist, der zu einem Aerosolerzeugungselement (170) bereitgestellt werden soll;
- das Aerosolerzeugungselement (170), das zum Erzeugen von Aerosolpartikeln und zum Einbringen derselben in den Aerosolstrom (114) eingerichtet ist;
- einen Aerosolschlauch (172), der zum Bereitstellen des die Aerosolpartikel enthaltenden Aerosolstroms (114) durch ein leitendes Element zum Patienten (116) eingerichtet ist; und
- ein Druckmodulationselement (174),
wobei das Druckmodulationselement (174) zum Bereitstellen eines zusätzlichen Drucks innerhalb des Aerosolstroms (114) zum Führen des Aerosolstroms (114) durch das leitende Element zum Patienten (116) eingerichtet ist,
**dadurch gekennzeichnet, dass** bei Verabreichung des Aerosols (112) an den Patienten (116) ein am Aerosolschlauch (172) herrschender Druck einen weiteren am Lufteinlass (164) herrschenden Druck um mindestens 0,01 mbar übersteigt.

2. Aerosolerzeugungsvorrichtung (162) nach dem vorhergehenden Anspruch, wobei das Aerosolerzeugungselement (170) aus einem Vernebler und/oder einem Pulvererzeuger ausgewählt ist.

3. Aerosolerzeugungsvorrichtung (162) nach einem der vorhergehenden Ansprüche, wobei das leitende Element ein Aerosolventil (154) und/oder eine Patientenschnittstelle (158) umfasst.

4. Aerosolerzeugungsvorrichtung (162) nach einem der vorhergehenden Ansprüche, wobei der Lufteinlass (164) zum Aufnehmen des Teils von Atemluft über eine Verbindungsstelle (167) aus dem Beatmungskreislauf (160) eingerichtet ist.

5. Aerosolerzeugungsvorrichtung (162) nach einem der vorhergehenden Ansprüche, wobei der zusätzliche Druck zum Verbessern einer Art des Führens des Aerosolstroms (114) zum Patienten (116) durch das leitende Element eingerichtet ist.

6. Aerosolerzeugungsvorrichtung (162) nach einem der vorhergehenden Ansprüche, wobei bei Verabreichung des Aerosols (112) an den Patienten (116) ein am Aerosolschlauch (172) herrschender Druck einen weiteren am Lufteinlass (164) herrschenden Druck um mindestens 0,1 mbar übersteigt.

7. Aerosolerzeugungsvorrichtung (162) nach einem der vorhergehenden Ansprüche, wobei das Druckmodulationselement (174) aus einem Ventilator, einem Gebläse, einer Pumpe, einem mechanischen Element und/oder einem elektromechanischen Element ausgewählt ist, die für diesen Zweck eingerichtet sind.

8. Aerosolerzeugungsvorrichtung (162) nach einem der vorhergehenden Ansprüche, wobei das Druckmodulationselement (174) ferner zum Begrenzen des zusätzlichen Drucks innerhalb der Aerosolerzeugungsvorrichtung (162) auf einen zusätzlichen Spitzendruck eingerichtet ist.

9. Aerosolerzeugungsvorrichtung (162) nach dem vorhergehenden Anspruch, wobei der zusätzliche Spitzendruck einen Wert von maximal 20 mbar oder darunter annimmt.

10. Aerosolerzeugungsvorrichtung (162) nach einem der zwei vorhergehenden Ansprüche, wobei das Druckmodulationselement (174) dazu eingerichtet ist, einen Volumenstrom von 0,01 l/min bis 3 l/min zu erzeugen, der im Vergleich zu dem Volumenstrom in einem Beatmungskreislauf dazu eingerichtet ist, eine unverdünnte Aerosolkonzentration direkt zum Patienten (116) zu transportieren.

11. Aerosolerzeugungsvorrichtung (162) nach einem der vorhergehenden Ansprüche, wobei die Aerosolerzeugungsvorrichtung (162) ferner einen Atemfilter (168) stromaufwärts des Aerosolerzeugungselements (170) umfasst, wobei der Atemfilter (168) zum Entfernen mindestens einer Störsubstanz aus der zu dem Aerosolerzeugungselement (170) bereitzustellenden Atemluft (166) eingerichtet ist.

12. Vorrichtung (110) zum Verabreichen eines Aerosols (112) an einen Patienten (116), umfassend:
- ein Aerosolventil (154), das zum Bereitstellen eines Aerosolstroms (114) zum Patienten (116) eingerichtet ist; und
- eine zum Erzeugen des Aerosolstroms (114) eingerichtete Aerosolerzeugungsvorrichtung (162) nach einem der auf die Aerosolerzeugungsvorrichtung (162) gerichteten vorhergehenden Ansprüche.

13. Vorrichtung (110) nach dem vorhergehenden Anspruch, die ferner eine Atmungsdetektionsvorrichtung (140) umfasst, wobei die Atmungsdetektionsvorrichtung (140) zum Bestimmen der Hebung (132) und/oder Zusammenziehung (134) des Brustkorbs (136) und/oder des Abdomens (138) des Patienten (116) in Bezug auf die Atmung des Patienten (116) eingerichtet ist.

14. Vorrichtung (110) nach dem vorhergehenden Anspruch, wobei die Atmungsdetektionsvorrichtung (140) Folgendes umfasst
- ein Detektionselement (142), das zum Aufzeichnen der Hebung (132) und/oder der Zusammenziehung (134) des Brustkorbs (136) und/oder des Abdomens (138) des Patienten (116) eingerichtet ist;
- ein Verarbeitungselement (146), das zum Bestimmen der Eingabedaten bezüglich des Atmungsmusters des Patienten (116) aus der Hebung (132) und/oder der Zusammenziehung (134) des Brustkorbs (136) und/oder des Abdomens (138) des Patienten (116) eingerichtet ist; und
- eine Kommunikationsschnittstelle (148), die zum Übertragen der Eingabedaten an die Verarbeitungsvorrichtung (120) eingerichtet ist.

15. Vorrichtung (110) nach dem vorhergehenden Anspruch, wobei das Detektionselement (142) ein Atemdetektionssensor ist, der aus einer Laufzeitkamera (144), einem Dehnungsmesselement, einem elektrischen Impedanztomografiesensor, einem Atmungsinduktivitäts-Plethysmographiesensor, einem Millimeterwellensensor, einem Radarsensor und/oder einem thermischen Sensor ausgewählt ist.

## Revendications

1. Dispositif générateur d'aérosol (162) configuré pour générer un flux d'aérosol (114) comprenant :
- une entrée d'air (164) configurée pour recevoir une partie d'air respirable (166) d'un circuit ventilatoire (160) à fournir à un élément générateur d'aérosol (170) ;
- l'élément générateur de l'aérosol (170) configuré pour générer des particules d'aérosol et les introduire dans le flux d'aérosol (114) ;
- un tube d'aérosol (172) configuré pour fournir le flux d'aérosol (114) comprenant les particules d'aérosol par l'intermédiaire d'un élément conducteur au patient (116) ; et
- un élément de modulation de pression (174),
dans lequel l'élément de modulation de pression (174) est configuré pour fournir une pression supplémentaire à l'intérieur du flux d'aérosol (114) pour guider le flux d'aérosol (114) à travers l'élément conducteur vers le patient (116),
**caractérisé en ce que**, lorsque l'aérosol (112) est administré au patient (116), une pression régnant dans le tube d'aérosol (172) dépasse d'au moins 0,01 mbar une autre pression régnant à l'entrée d'air (164).

2. Dispositif générateur d'aérosol (162) selon la revendication précédente, dans lequel l'élément générateur d'aérosol (170) est choisi parmi au moins un nébuliseur ou un générateur de poudre.

3. Dispositif générateur d'aérosol (162) selon l'une quelconque des revendications précédentes, dans lequel l'élément conducteur comprend au moins l'un d'une valve d'aérosol (154) ou d'une interface patient (158).

4. Dispositif générateur d'aérosol (162) selon l'une quelconque des revendications précédentes, dans lequel l'entrée d'air (164) est configurée pour recevoir la partie d'air respirable par le biais d'une jonction (167) à partir du circuit de ventilation (160).

5. Dispositif générateur d'aérosol (162) selon l'une quelconque des revendications précédentes, dans lequel la pression supplémentaire est conçue pour améliorer une manière de guider le flux d'aérosol (114) vers le patient (116) par l'intermédiaire de l'élément conducteur.

6. Dispositif générateur d'aérosol (162) selon l'une quelconque des revendications précédentes, dans lequel, lorsque l'aérosol (112) est administré au patient (116), une pression régnant dans le tube d'aérosol (172) dépasse d'au moins 0,1 mbar une autre pression régnant à l'entrée d'air (164).

7. Dispositif générateur d'aérosol (162) selon l'une quelconque des revendications précédentes, dans lequel l'élément de modulation de pression (174) est choisi parmi au moins l'un d'un ventilateur, d'un respirateur, d'une pompe, d'un élément mécanique ou d'un élément électromécanique configuré à cet effet.

8. Dispositif générateur d'aérosol (162) selon l'une quelconque des revendications précédentes, dans lequel l'élément de modulation de pression (174) est en outre configuré pour limiter la pression supplémentaire à l'intérieur du dispositif générateur d'aérosol (162) à une pression de crête supplémentaire.

9. Dispositif générateur d'aérosol (162) selon la revendication précédente, dans lequel la pression de crête supplémentaire prend une valeur égale ou inférieure à 20 mbar au maximum.

10. Dispositif générateur d'aérosol (162) selon l'une quelconque des deux revendications précédentes, dans lequel l'élément de modulation de pression (174) est configuré pour générer un débit volumique de 0,01 L/min à 3 L/min, lequel, comparé au débit volumique dans un circuit de ventilation, est configuré pour transporter directement une concentration d'aérosol non dilué vers le patient (116).

11. Dispositif générateur d'aérosol (162) selon l'une quelconque des revendications précédentes, dans lequel le dispositif générateur d'aérosol (162) comprend en outre un filtre respiratoire (168) en amont de l'élément générateur d'aérosol (170), dans lequel le filtre respiratoire (168) est configuré pour éliminer au moins une substance perturbatrice de l'air respirable (166) à fournir à l'élément générateur d'aérosol (170).

12. Appareil (110) d'administration d'un aérosol (112) à un patient (116) comprenant :
- une valve d'aérosol (154) configurée pour fournir un flux d'aérosol (114) au patient (116) ; et
- un dispositif générateur d'aérosol (162) configuré pour générer le flux d'aérosol (114) selon l'une quelconque des revendications précédentes dirigé vers le dispositif générateur d'aérosol (162).

13. Appareil (110) selon la revendication précédente, comprenant en outre un dispositif de détection respiratoire (140), dans lequel le dispositif de détection respiratoire (140) est configuré pour déterminer au moins l'une de l'élévation (132) ou de la constriction (134) d'au moins l'un de la poitrine (136) ou de l'abdomen (138) du patient (116) en lien avec la respiration du patient (116).

14. Appareil (110) selon la revendication précédente, dans lequel le dispositif de détection respiratoire (140) comprend
- un élément de détection (142) configuré pour enregistrer au moins l'une de l'élévation (132) ou de la constriction (134) de la poitrine (136) et/ou de l'abdomen (138) du patient (116) ;
- un élément de traitement (146) configuré pour déterminer les données d'entrée liées au schéma de respiration du patient (116) à partir de l'élévation (132) et/ou de la constriction (134) de la poitrine (136) et/ou de l'abdomen (138) du patient (116) ; et
- une interface de communication (148) configurée pour transmettre les données d'entrée au dispositif de traitement (120).

15. Appareil (110) selon la revendication précédente, dans lequel l'élément de détection (142) est un capteur de détection de respiration choisi parmi au moins l'un d'une caméra de temps de vol (144), d'un élément à jauge de contrainte, d'un capteur tomographique à impédance électrique, d'un capteur pléthysmographique inductif respiratoire, d'un capteur à ondes millimétriques, d'un capteur radar ou d'un capteur thermique.
